# EUROPEAN PATENT SPECIFICATION

(11) **EP 4 196 055 B1**
(45) Date of publication and mention of the grant of the patent: **30.04.2025**
(21) Application number: 21856973.9
(22) Date of filing: 24.06.2021
(51) Int. Cl.: A61F 2/24, D03D 27/08

(54) **A TEXTILE BASED CARDIAC PATCH FOR MYOCARDIUM REGENERATION**
HERZPFLASTER AUF TEXTILBASIS ZUR REGENERATION DES HERZMUSKELS
TIMBRE CARDIAQUE À BASE DE TEXTILE POUR LA RÉGÉNÉRATION DU MYOCARDE

(30) Priority: 17.08.2020 TR 202012929
(43) Date of publication of application: 21.06.2023
(73) Proprietor: Erciyes Universitesi, 38039 Kayseri (TR); Boyteks Tekstil Sanayi Ve Ticaret A.s., Hacilar/Kayseri (TR)
(72) Inventor: HAROGLU, Derya, Kayseri (TR); EKEN, Ahmet, Melikgazi/Kayseri (TR); ILBEYLI, Hakan, 38070 Kayseri (TR); GONEN, Zeynep Burcin, Melikgazi/Kayseri (TR); BAGDAS, Hakan, 38070 Kayseri (TR)
(74) Representative: Dericioglu, E. Korhan
(86) International application number: PCT/TR2021/050649
(87) International publication number: WO 2022/039693

(56) References cited:
- US-A1- 2004 176 658
- TANG JUNNAN, VANDERGRIFF ADAM, WANG ZEGEN, HENSLEY MICHAEL TAYLOR, CORES JHON, ALLEN TYLER A., DINH PHUONG-UYEN, ZHANG JINYING, CA: "A Regenerative Cardiac Patch Formed by Spray Painting of Biomaterials onto the Heart", TISSUE ENGINEERING. PART C, vol. 23, no. 3, 1 March 2017 (2017-03-01), US , pages 146 - 155, XP055908056, ISSN: 1937-3384, DOI: 10.1089/ten.tec.2016.0492
- WALSH, ROBERT G: "Design and features of the acorn corcap tm cardiac support device: the concept of passive mechanical diastolic support.", HEART FAILURE REVIEWS, vol. 10, no. 2, 2005, pages 101 - 107, XP019207221, DOI: 10.1007/s10741-005-4637-x
- CHEN, JIYANG: "A Biodegradable Knitted Cardiac Patch for Myocardium Regeneration using Cardiosphere-derived Cells (CDCs).", JOURNAL OF DONGHUA UNIVERSITY, vol. 34, no. 2, 2017, pages 310 - 315, XP055908059
- NIKOLOV ET AL.: "Development of a 3D model of terry fabric.", INTERNATIONAL JOURNAL OF CLOTHING SCIENCE AND TECHNOLOGY, vol. 24, no. 4, 2012, pages 237 - 250, XP055908066

## Description

### Field of the Invention

The present invention relates to a textile based cardiac patch for myocardium regeneration for use in the field of cardiac tissue engineering. In addition, the present invention can also be used in tissue engineering and regenerative medicine.

### Background of the Invention

According to the World Health Organization (WHO) report, worldwide more than 9 million people died from coronary artery disease (CAD) in 2016. This number corresponds to approximately 53% of all deaths from cardiovascular diseases (CVDs) (WHO, 2017). Cardiovascular disease (CVD) is the primary cause of all deaths worldwide (WHO, 2017). Myocardial infarction (MI), heart attack, is the primary health problem for people with coronary artery disease (CAD).

Myocardial reperfusion therapy (fibrinolysis or balloon angioplasty and/or placing a stent), which is recommended within the first 90 minutes after a heart attack, is performed as a routine treatment to restore coronary blood flow associated with a heart attack, preserve viable myocytes, and recover ventricular function (Dalal et al., 2013). Myocardial reperfusion itself can cause damage to the heart resulting in further cardiomyocyte loss (Simonis, Strasser, & Ebner, 2012; Seropian, Toldo, Van Tassell, & Abbate, 2014). Mechanical treatments (LVADs, TAHs) can cause complications such as post-operation bleeding in patients, right heart failure, stroke and infection (Givertz, 2011). Even though heart transplantation can be performed in end-stage cardiac patients, the limited number of organ donors and potential immune complications (rejection, tumor formation) after transplantation lead scientists to search for new solutions (Mondal, 2012; Patra, Boccaccini, & Engel, 2015).

Clinical studies have shown that ventricular dilatation and mortality are reduced through long-term pharmacological (angiotensin-converting enzyme inhibitors (ACEi), β-adrenergic-receptor blockers) or mechanical (left ventricular assist devices (LVADs), cardiac resynchronization therapy, total artificial hearts (TAHs)) therapies after acute myocardial infarction (Koitabashi & Kass, 2012; Galli & Lombardi, 2016). The left ventricular assist devices (LVADs) or total artificial hearts (TAHs) are implanted to end-stage cardiac patients as lifesavers before heart transplantation (Givertz, 2011).

CorCapTM cardiac assist device (CSD) (Acorn Cardiovascular Inc., US) (Lilip L, et al. US patent 6682474 B2, Jan.27, 2004) was designed to reduce myocardial wall stress and prevent further ventricular dilatation in patients with chronic heart failure. The said device (CSD) is a warp-knitted mesh made of poly(ethylene) terephthalate (PET) multifilament yarns and it is wrapped around the heart surface (Walsh, 2005). CorCapTM cardiac assist device is not yet approved by The U.S. Food and Drug Administration (FDA).

Cell-based therapies aim to restore the function of the heart and/or regenerate the heart by injecting cells (mononuclear bone marrow cells, embryonic stem cells, mesenchymal stem cells, skeletal muscle cells, endothelial progenitor cells, induced pluripotent stem cells) directly into the infarcted area (Zammaretti & Jaconi, 2004). The main disadvantage of cell-based therapies is that the survival rate of successfully injected cells is very low (about 90% of cells die within a week) (Malliaras & Marbán, 2011).

Cell sheet and decellularized matrix technologies are being studied to improve cell therapy (Mayorga et al., 2019). In cell sheet technology, cell sheets are obtained by adding sheet by sheet from cell culture, the surface of which is coated with a temperature sensitive polymer (poly(N-isopropylacrylamide)), while preserving their own extracellular matrix (ECMs) (Masuda et al, 2007; Takahashi et al., 2019) . The three-dimensional cell sheets that are created can be implanted in the infarcted area of the heart (Yoshikawa et al., 2018). Decellularization technology aims to decellularize tissues or organs with various mechanical, chemical, and enzymatic techniques while preserving the natural ECM and vascular structure (Gilbert et al., 2006; Keane et al., 2015). It is needed to study more to reach transplantable structures in the field of decellularized matrix technology (Gilbert et al., 2006; Rodrigues et al., 2018).

An alternative strategy for myocardial tissue regeneration is to implant the structure called 'cardiac patch' on the damaged area of the cardiac muscle (Chen, Harding, Ali, Lyon, & Boccaccini, 2008; Vunjak-Novakovic et al., 2009). In the literature, the expression "a three-dimensional (3D) biocompatible material with or without growth factor, with or without cells" is used for the definition of cardiac patch (Kitsara, M., et al., 2017). Cardiac patch scaffold is defined as "three-dimensional biocompatible and/or biodegradable material". Cells are seeded on a three-dimensional biocompatible scaffold (Leor & Cohen, 2004; Vunjak-Novakovic et al., 2009). The scaffold temporarily hosts cells until the cells form their own extracellular matrix, providing the necessary mechanical stability (Leor & Cohen, 2004; Vunjak-Novakovic et al., 2009). The cell-seeded scaffold (cardiac patch) promotes cell growth and differentiation by mimicking the natural myocardium after implantation in the infarcted area of the heart (Leor & Cohen, 2004; Chen et al., 2008). Textile engineering offers various engineered structures such as knitting or electrospinning to promote cell growth and proliferation by mimicking the mechanical properties of natural myocardium. Various biocompatible and/or biodegradable natural, synthetic, and a mixture of natural and synthetic biomaterials have been used to manufacture scaffolds until today (Rodriges, I. C. P. et al., 2018). Even though natural materials (collagen, fibrin, alginate) increase cell adhesion and proliferation due to the fact that they are of natural origin, their mechanical properties and reproducibility are poor (Feiner, R. et al., 2019). On the other hand, synthetic biomaterials (polyethylene terephthalate (PET), poly(lactic acid) (PLA), poly(glycolic acid) (PGA), poly(ε-caprolacton) (PCL), poly(glycerol sebacate) (PGS)) are similar to the cardiac muscle, and they are mechanically assisting. However, biodegradable ones need to be developed further in order to be able to control their degradation mechanisms and maintain their elasticity in the long term (Chen et al., 2008).

Boublik et al. (2005) performed studies on seeding newborn rat heart cells on a two-layer knitted jersey fabric made of hyaluronan benzyl ester (Hyaff-11). In these studies, it was observed that when the cyclic stretch test was applied for a week in culture made in the laboratory environment, the stiffness of the structure was maintained, however the final tensile strength decreased. However, in the said study there is no structure exactly mimicking the cardiac muscle.

Ayaz et al. (2014) produced thermoplastic polycarbonate-urethane (PCU, Bionate^{®}) and poly(L-lactide-co-glycolide) (PLGA) scaffolds by electrospinning method. In this study, polyester (PE) and cotton (CT) weft knitted fabric (jersey) was placed on a flat metal collector in an electrospinning device. Therefore, it was desired to obtain polycarbonate-urethane (PCU, Bionate^{®}) and poly(L-lactide-co-glycolide) (PLGA) scaffolds having knitted fabric topologies placed on the collector. Newborn rat cardiomyocytes were seeded on scaffolds in laboratory environment (Lelkes P I, et al. US patent 9,668,846, June 6, 2017). It is claimed in this study that the elastic modulus (approximately 750 kPa (0.75 Mpa)) of the cardiac patch produced has mechanical properties similar to that of the human left ventricle at the end of systole. However, this value is quite high for cardiac muscle.

Chen et al. (2008) claim that the stiffness value of the cardiac muscle at the end of diastole (15-22% strain) rises up to 50 kPa in healthy hearts and up to 200-300 kPa in hearts with chronic heart disease.

Chen (2015) examined cardiac patches weft knitted from multifilament polylactic acid (PLA) yarns. In the study, cardiosphere-derived stem cells (CDC) from dogs were seeded on patch fabrics in a laboratory environment. It was observed that the rib stitch structure best mimicked the human myocardium in terms of cell proliferation and mechanical properties. In the said study, the stress values in the weft and warp directions (between 4 and 6 kPa) for 20% strain of the rib stitch structure knitted from polyester were lower than that of normal cardiac muscle (approximately 10 kPa).

Prabhakaran et al. (2011) reported that poly(L-lactide-co-glycolide)/gelatin (PLGA)/Gel) cardiac patch produced with electrospinning method exhibited superior cell proliferation, cell integration, and mechanical support compared to PLGA cardiac patch produced by electrospinning method. In this study, cardiomyocytes obtained from rabbits were seeded on nanofiber scaffolds in a laboratory environment. The elastic modulus of the scaffolds produced in the said study was obtained as PLGA: 80 MPa; PLGA/Gel: 25.12 MPa.

In the literature review, no ideal scaffold with mechanical properties similar to cardiac muscle (scaffold) was found. A healthy cardiac muscle has a stiffness (or Young's modulus) of about 10-20 kPa when the strain is less than 10% (at the beginning of diastole) (Chen et al., 2008). The stiffness value at the end of diastole (15-22% strain) rises up to 50 kPa in healthy hearts and up to 200-300 kPa in hearts with chronic heart disease (Chen et al., 2008).

In the United States patent application no. US2004176658, the invention is related to warp knit fabrics useful for medical articles and methods of making same. The present invention provides articles useful in medical applications including the treatment of heart diseases, and methods for producing the articles. Embodiments include warp knitted fabrics, both single and multilayer, medical articles and methods of making the same.

### Summary of the Invention

The objective of the present invention is to provide a textile-based cardiac patch for myocardium regeneration. For this purpose, within the scope of the invention, terry fabrics with mechanical properties similar to the cardiac muscle have been developed by using biocompatible yarn as scaffold material. Terry fabrics are divided into two as woven and knitted terry fabrics. There may be pile loops on only one surface of terry fabrics, as well as there may be pile loops on both surfaces. The terry fabric becomes three dimensional (3D) with the said pile loops provided on the fabric surface. Even though terry fabric is a technology known in home textiles such as towel and undersheet production, no publication and/or patent has been found in the technical field for the use of three-dimensional terry fabric as a cardiac patch.

Another objective of the present invention is to provide a textile-based cardiac patch product which exhibits similar performance to ideal cardiac muscle. Considering that the stiffness value (Young's modulus) of the ideal cardiac muscle at the end of diastole (strain 15-22%) rises up to 50 kPa in healthy hearts and up to 200-300 kPa in hearts with chronic heart disease; it has been observed in the studies performed with terry fabric that a stiffness value of less than 300 kPa can be achieved in the invention for 20% strain in the weft and warp directions. The three-dimensional (3D) porous structure of terry fabric creates an ideal environment for cell attachment, growth, and proliferation. The terry fabric provides an additional surface area for cell migration and attachment throughout the fabric thickness.

The three-dimensional terry fabric used in the invention provides various advantages for its intended use. The said advantages are summarized below:
- An additional surface area is obtained by means of the pile loops on the surface of the terry fabric.
- Thanks to the pile loops on the surface of the terry fabric, an additional pore structure is provided, being different from the studies made with only base fabric in the state of the art.
- A structure whose mechanical properties are close to the mechanical properties of cardiac muscle can be obtained.
- Yarns with different properties can be used for base and pile loops as long as they do not create a problem in terms of manufacturability.

### Detailed Description of the Invention

The present invention relates to a cardiac patch for use in tissue engineering, regenerative medicine and/or myocardium regeneration. The said cardiac patch comprises three-dimensional terry fabrics as scaffold material. Terry fabrics in cardiac patch can be produced with synthetic and natural yarns in different numbers and different filament numbers. Three-dimensional (3D) terry fabrics to be used in cardiac patch production in the invention are produced from biocompatible and/or biodegradable materials. The said terry fabrics must have a porous structure in order for the cells to grow and proliferate.

The said terry fabrics can be
- Knitted and/or woven terry fabrics (terry woven fabrics, terry knitted fabrics, pile-loop knit fabrics)
- Cut-pile loop knitted and/or woven terry fabrics (woven velour fabrics, knitted velour fabrics, cut-pile loop knit fabrics),
- Terry fabric structures from synthetic (such as Polyethylene terephthalate (PET) and poly(lactic acid) (PLA)) and natural (such as cotton and silk) materials.

Terry machines are used in production process of terry fabrics. For this, twisted or intermingled synthetic and/or natural yarns in different numbers and different filament numbers can be used. In one embodiment of the invention, twisted yarns may be used for base loops and intermingled yarns may be used for pile loops. Likewise, it is possible to use synthetic yarn for base loops and natural yarn for pile loops. However, it is important that the use of the said different yarns is done in a way that allows the production of terry fabric. Depending on the yarn properties, their manufacturability as terry fabric should be tested.

The mechanical properties of the terry fabric being able to mimic the mechanical properties of the cardiac muscle is important in terms of use in tissue engineering, regenerative medicine and/or myocardium regeneration. In accordance with this purpose, it is known that knitted fabrics technically and inherently have high elasticity. Furthermore, it is also possible to increase this elasticity with machine settings and finishing processes. In addition, in regenerative medicine, woven terry fabric can be used as scaffold material in cases where higher stiffness (Young's modulus) is required.

Then, finishing processes (washing, drying, thermofixing, etc.) can be applied to the fabric produced in terry machines. In the production process, while terry fabric machines can be used, current suitable machinery can also be used by customizing them. It is possible to produce fabrics with different properties by changing the yarn, machine and finishing process parameters.

C2C12 cells were seeded on the produced biocompatible anisotropic cardiac patch scaffolds (terry fabric scaffolds). It was observed by 3-(4,5-dimethylthiazol-2-yl)-2,5-diphenyltetrazolium bromide (MTT) assay that the cells survived and proliferated in scaffolds . MTT assay is a routine method known in state of the art which is used to measure cell viability and proliferation in tissue engineering scaffolds.

## Claims

1. A cardiac patch for use in regenerative medicine and/or myocardium regeneration, **characterized in that** it comprises a three-dimensional terry fabric with a porous structure, made of biocompatible and/or biodegradable material, as the scaffold material.

2. A cardiac patch according to claim 1, **characterized in that** it comprises three-dimensional knitted terry fabric as the scaffold material.

3. A cardiac patch according to claim 1, **characterized in that** it comprises three-dimensional woven terry fabric as the scaffold material.

4. A cardiac patch according to claim 1, **characterized in that** it comprises three-dimensional terry fabric made from synthetic materials as the scaffold material.

5. A cardiac patch according to claim 1, **characterized in that** it comprises three-dimensional terry fabric made from natural materials as the scaffold material.

## Patentansprüche

1. Ein Herzpflaster zur Verwendung in der regenerativen Medizin und/oder der Myokardregeneration, **dadurch gekennzeichnet, dass** es ein dreidimensionales Frotteegewebe mit einer porösen Struktur aus biokompatiblem und/oder biologisch abbaubarem Material als Gerüstmaterial umfasst.

2. Ein Herzpflaster nach Anspruch 1, **dadurch gekennzeichnet, dass** es ein dreidimensionales gestricktes Frotteegewebe als Gerüstmaterial umfasst.

3. Ein Herzpflaster nach Anspruch 1, **dadurch gekennzeichnet, dass** es ein dreidimensionales gewebtes Frotteegewebe als Gerüstmaterial umfasst.

4. Ein Herzpflaster nach Anspruch 1, **dadurch gekennzeichnet, dass** es dreidimensionales Frotteegewebe aus synthetischen Materialien als Gerüstmaterial umfasst.

5. Ein Herzpflaster nach Anspruch 1, **dadurch gekennzeichnet, dass** es dreidimensionales Frotteegewebe aus natürlichen Materialien als Gerüstmaterial umfasst.

## Revendications

1. Un patch cardiaque destiné à la médecine régénérative et/ou à la régénération du myocarde, **caractérisé en ce qu'**il comprend un tissu éponge en trois dimensions à structure poreuse, constitué d'un matériau biocompatible et/ou biodégradable, comme matériau d'échafaudage.

2. Un patch cardiaque selon la revendication 1, **caractérisé en ce qu'**il comprend un tissu éponge tricoté en trois dimensions comme matériau d'échafaudage.

3. Un patch cardiaque selon la revendication 1, **caractérisé en ce qu'**il comprend un tissu éponge tissé en trois dimensions comme matériau d'échafaudage.

4. Un patch cardiaque selon la revendication 1, **caractérisé en ce qu'**il comprend un tissu éponge en trois dimensions à partir de matériaux synthétiques comme matériau d'échafaudage.

5. Un patch cardiaque selon la revendication 1, **caractérisé en ce qu'**il comprend un tissu éponge en trois dimensions à partir de matériaux naturels comme matériau d'échafaudage.
